# EUROPEAN PATENT SPECIFICATION

(11) **EP 1 165 071 B1**
(45) Date of publication and mention of the grant of the patent: **23.11.2005**
(21) Application number: 00918435.9
(22) Date of filing: 27.03.2000
(51) Int. Cl.: A61K 31/4188, A61P 35/00

(54) **IMPROVED CANCER TREATMENT WITH TEMOZOLOMIDE**
VERBESSERTE KREBSBEHANDLUNG MIT TEMOZOLOMID
TRAITEMENT DU CANCER AMELIORE AVEC LE TEMOZOLOMIDE

(30) Priority: 30.03.1999 US 281348
(43) Date of publication of application: 02.01.2002
(73) Proprietor: SCHERING CORPORATION, Kenilworth, New Jersey 07033-0530 (US)
(72) Inventor: RAGAB, Mohamed, H., Westfield, NJ 07090 (US)
(74) Representative: UEXKÜLL & STOLBERG
(86) International application number: PCT/US2000/008079
(87) International publication number: WO 2000/057867

(56) References cited:
- WO-A-97/12630
- DATABASE WPI Section Ch, Week 199828 Derwent Publications Ltd., London, GB; Class B02, AN 1998-189166 XP002156886 & CA 2 184 545 A (SCHERING CORP), 1 February 1998 (1998-02-01)
- DATABASE WPI Section Ch, Week 199828 Derwent Publications Ltd., London, GB; Class B02, AN 1998-189167 XP002156887 & CA 2 184 546 A (SCHERING CORP), 1 February 1998 (1998-02-01)
- BROCK CATHRYN S ET AL: "Phase I trial of temozolomide using an extended continuous oral schedule." CANCER RESEARCH, vol. 58, no. 19, 1 October 1998 (1998-10-01), pages 4363-4367, XP000971768 ISSN: 0008-5472
- WEDGE STEPHEN R ET AL: "Effect of single and multiple administration of an O-6-benzylguanine/temozolomide combination: An evaluation in a human melanoma xenograft model." CANCER CHEMOTHERAPY AND PHARMACOLOGY, vol. 40, no. 3, 1997, pages 266-272, XP000978236 ISSN: 0344-5704
- NEWLANDS E S ET AL: "TEMOZOLOMIDE: A REVIEW OF ITS DISCOVERY, CHEMICAL PROPERTIES, PRE-CLINICAL DEVELOPMENT AND CLINICAL TRIALS" CANCER TREATMENT REVIEWS,US,SAUNDERS, vol. 23, no. 1, 1997, pages 35-61, XP000921344 ISSN: 0305-7372
- NICHOLSON H STACY ET AL: "Phase I study of temozolomide in children and adolescents with recurrent solid tumors: A report from the Children's Cancer Group." JOURNAL OF CLINICAL ONCOLOGY, vol. 16, no. 9, 1998, pages 3037-3043, XP000978248 ISSN: 0732-183X

## Description

This invention relates to the treatment of cancer and in particular to the treatment of cancers with Temozolomide.

### BACKGROUND OF THE INVENTION

Temozolomide is known for its anti-tumor effects. For example, in one study clinical responses were achieved in 17% of patients having advanced melanoma (Newlands et al. Br. J. Cancer 65 (2) 287-291 (1992)). In another study, a clinical response was achieved in 21% of patients with advanced melanoma (Journal of Clinical Oncology, Vol 13, No. 4 (April), 1995, pp 910-913). Treatment of gliomas in adults with temozolomide is also known (Eur. J. Cancer 1993; 29A:940). Treatment of the following cancers in adults with temozolomide has also been disclosed: metastatic melanoma; high grade glioma, glioblastoma and other brain cancers; lung cancer; breast cancer; testicular cancer; colon and rectal cancers; carcinomas; sarcomas; lymphomas; leukemias; and mycosis fungoides. Prior to the present invention, the generally accepted method for administering temozolomide was to administer it over a 28 day cycle, in which it is administered daily for the first 5 days of the cycle, followed by 23 days of rest, in which it is not administered. Newlands et al., Br. J. Cancer 65 (2) 287-291 (1992). A clinical trial has also been carried out wherein temozolomide was administered continuously as a daily dose for 6-7 weeks in conjunction with radiation treatment. See, e.g., Brock et al., Cancer Research 58, 4363-4367 (1998).

### SUMMARY OF THE INVENTION

The present invention is directed to the use of temozolomide for the preparation of a composition for treating a patient afflicted with cancer comprising administration of this composition to said patient for at least two cycles of a cyclical dosing schedule, wherein each cycle comprises a dosing period of 5 to 25 days, in which temozolomide is administered daily, at a dose of 40 to 150 mg/m²/day, followed by a rest period of 5 to 14 days in which temozolomide is not administered.

### DETAILED DESCRIPTION

The term "temozolomide" is intended to mean a compound having the formula: 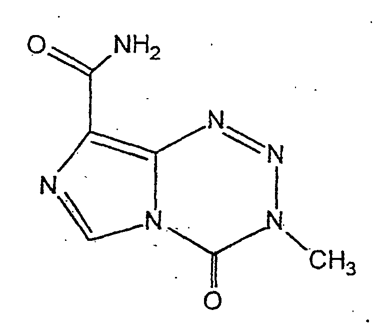 One chemical name for temozolomide is 3,4-dihydro-3-methyl-4-oxoimidazo-[5,1-d]1,2,3,4-tetrazin-8-carboximide. The synthesis of temozolomide is well known. See, for example, Stevens et al., J. Med. Chem, 1984, 27, 196-201 and Wang et al., J. Chem. Soc., Chem. Commun., 1994, pp 1687-1688.

As used herein, the term "mg/m²/day" refers to a daily dose measured in milligrams per square meter of body surface area of the patient.

As used herein, the term "patient" refers to a mammal, preferably a human.

Examples of cancers treatable by this invention include, but are not limited to melanoma; high grade glioma, glioblastoma and other brain cancers; lung cancer; breast cancer; testicular cancer; gastro intestinal cancers including colon, rectal, pancreatic, and gastric cancers, hepatocellular carcinoma; head and neck cancers; prostate cancer, renal cell carcinoma; adenocarcinoma; sarcomas; lymphomas; leukemias; and mycosis fungoides. This invention contemplates treating these cancers and other cancers at any stage from the discovery of the cancer to the advanced stage. The invention includes treatment of the primary cancer and metastases thereof.

A person afflicted with cancer may exhibit one or more of the following signs or symptoms:
(a) presence of cancerous tumor,
(b) fatigue,
(c) pain,
(d) decreased performance status from tumor burden, and
(e) the well known symptoms associated with each specific cancer.

The rest period according to the present invention (the portion of the cycle in which temozolomide is not administered) is 5 to 14 days, more preferably, 5 to 10 days, most preferably, 1 week. The dosing period according to the present invention is 5 to 25 days, more preferably, 1, 2, or 3 weeks, most preferably 1 or 3 weeks. The treatment cycles may be continued for as long as needed to cause the cure, remission, or elimination of the cancer that is being treated.

The daily dose during the dosing period of the present invention is 40 to 150 mg/m²/day, more preferably 40 to 125 mg/m²/day, most preferably 75 to 125 mg/m²/day. The daily dose may be administered as a single dose, or as multiple doses adding up to the single dose. For example, a daily dose of 100 mg/m² may be administered as two doses of 50 mg/m², or four doses of 25 mg/m². The selected dosage may be decreased, if intolerable side effects or hematologic toxicity are encountered.

A common, but tolerable side effect of temozolomide is nausea and vomiting. This can be alleviated by administering an anti-emetic in conjunction with the temozolomide. It is preferred that the anti-emetic Ondansetron be given p.o. in a dose of about 8 mg about 30 minutes before temozolomide administration. Other anti-emetics such as Hasaldol, Benadryl, and Ativan may also be used as needed.

Temozolomide is preferably administered orally in capsule form wherein it is admixed with conventional pharmaceutical carriers. Preferred temozolomide capsule formulations are:

| Ingredient | mg/Capsule | | | |
|---|---|---|---|---|
| temozolomide | 5 | 20 | 100 | 250 |
| Anhydrous Lactose NF | 132.8 | 182.2 | 175.7 | 154.3 |
| Sodium Starch Glycolate NF | 7.5 | 11.0 | 15.0 | 22.5 |
| Colloidal Silicon Diozide NF | 0.2 | 0.2 | 0.3 | 0.7 |
| Tartaric Acid NF | 1.5 | 2.2 | 3.0 | 9.0 |
| Steric Acid NF | 3.0 | 4.4 | 6.0 | 13.5 |
| Capsule Size* | 3 | 2 | 1 | 0 |

| | | | | |
|---|---|---|---|---|
| * White opaque, preservative-free, two-piece hard gelatin capsules | | | | |

Other forms of administration of temozolomide, as they become available, are contemplated, such as by IV injection or infusion, intrathecally, by sustained release dosage form, syrup, suppository, transdermal, nasal spray, etc.. Any form of administration will work so long as the proper dosage is delivered without destroying the temozolomide.

It may be preferable in some instances to administer an initial large oral bolus dose of about 100 to 500 mg/m² prior to beginning the cyclical dosing regimen of the present invention.

The medical kit in accordance with the present invention may be in any form suitable for providing a supply of temozolomide for at least one cycle, together with written instructions for administering it according to the cyclical dosing schedule. Examples include, but are not limited to, various containers (e.g., bottles, cartons, blister packs, and ampules) either accompanied by a package insert describing the cyclical dosing instructions, or wherein the cyclical dosing instructions are printed on, or affixed to the container.

The following examples illustrate the foregoing invention, although such examples should not be construed as limiting the scope of the invention.

### EXAMPLE 1

To a patient suffering from glioma, administer temozolomide for a period of twelve 14-day cycles, each cycle consisting of a one week period in which temozolomide is administered at the rate of 100 mg/m²/day, followed by a one week rest period in which temozolomide is not administered.

### EXAMPLE 2

To a patient suffering from glioma, administer temozolomide for a period of six 28-day cycles, each cycle consisting of a three week period in which temozolomide is administered at the rate of 100 mg/m²/day, followed by a one week rest period in which temozolomide is not administered.

### EXAMPLE 3

To a patient suffering from advanced melanoma, administer temozolomide for a period of twelve 14-day cycles, each cycle consisting of a one week period in which temozolomide is administered at the rate of 100 mg/m²/day, followed by a one week rest period in which temozolomide is not administered.

### EXAMPLE 4

To a patient suffering from advanced melanoma, administer temozolomide for a period of six 28-day cycles, each cycle consisting of a three week period in which temozolomide is administered at the rate of 100 mg/m²/day, followed by a one week rest period in which temozolomide is not administered.

While the present invention has been described in conjunction with the specific embodiments set forth above, many alternatives, modifications and variations thereof will be apparent to those of ordinary skill in the art. All such alternatives, modifications and variations are intended to fall within the spirit and scope of the present invention.

## Claims

1. Use of temozolomide for the preparation of a composition for treating a patient afflicted with cancer wherein the composition is for administration to said patient for at least two cycles of a cyclical dosing schedule, wherein each cycle comprises a dosing period of 5 to 25 days, in which temozolomide is for administration daily, at a dose of 40 to 150 mg/m²/day, followed by a rest period of 5 to 14 days in which temozolomide is not administered.

2. Use according to claim 1, wherein the rest period is 5 to 10 days.

3. Use according to claim 2, wherein the daily dose is 75 to 125 mg/m²/day.

4. Use according to claim 1, wherein the rest period is one week.

5. Use according to claim 4, wherein the daily dose is 75 to 125 mg/m²/day.

6. Use according to claim 1, wherein the dosing period is one, two, or three weeks.

7. Use according to claim 6, wherein the rest period is one week.

8. Use according claim 7, wherein the dosing period is one week.

9. Use according to claim 8, wherein the daily dose is 75 to 125 mg/m²/day.

10. Use according to claim 7, wherein the dosing period is three weeks.

11. Use according to claim 10, wherein the daily dose is 75 to 125 mg/m²/day.

## Patentansprüche

1. Verwendung von Temozolomid zur Herstellung einer Zusammensetzung zur Behandlung eines Patienten, der unter Krebs leidet, wobei die Zusammensetzung zur Verabreichung an den Patienten für mindestens zwei Zyklen eines zyklischen Dosierungsplans ist, wobei jeder Zyklus einen Dosierungszeitraum von 5 bis 25 Tagen umfasst, in welchem Temozolomid für die tägliche Verabreichung in einer Dosis von 40 bis 150 mg/m²/Tag ist, gefolgt von einem Ruhezeitraum von 5 bis 14 Tagen, in dem Temozolomid nicht verabreicht wird.

2. Verwendung nach Anspruch 1, bei der der Ruhezeitraum 5 bis 10 Tage ist.

3. Verwendung nach Anspruch 2, bei der die tägliche Dosis 75 bis 125 mg/m²/Tag ist.

4. Verwendung nach Anspruch 1, bei der der Ruhezeitraum eine Woche ist.

5. Verwendung nach Anspruch 4, bei der die tägliche Dosis 75 bis 125 mg/m²/Tag ist.

6. Verwendung nach Anspruch 1, bei der der Dosierungszeitraum eine, zwei oder drei Wochen ist.

7. Verwendung nach Anspruch 6, bei der der Ruhezeitraum eine Woche ist.

8. Verwendung nach Anspruch 7, bei der der Dosierungszeitraum eine Woche ist.

9. Verwendung nach Anspruch 8, bei der die tägliche Dosis 75 bis 125 mg/m²/Tag ist.

10. Verwendung nach Anspruch 7, bei der der Dosierungszeitraum drei Wochen ist.

11. Verwendung nach Anspruch 10, bei der die tägliche Dosis 75 bis 125 mg/m²/Tag ist.

## Revendications

1. Emploi du temozolomide dans la préparation d'une composition destinée au traitement d'un patient atteint d'un cancer, laquelle composition est conçue pour être administrée audit patient pendant au moins deux cycles d'un schéma posologique cyclique, chaque cycle comportant une période de traitement de 5 à 25 jours, au cours de laquelle on administrera chaque jour du temozolomide au patient, en une dose de 40 à 150 mg/m²/jour, suivie d'une période de repos de 5 à 14 jours, au cours de laquelle on n'administrera pas de temozolomide.

2. Emploi conforme à la revendication 1, dans lequel la période de repos dure de 5 à 10 jours.

3. Emploi conforme à la revendication 2, dans lequel la dose quotidienne vaut de 75 à 125 mg/m²/jour.

4. Emploi conforme à la revendication 1, dans lequel la période de repos dure une semaine.

5. Emploi conforme à la revendication 4, dans lequel la dose quotidienne vaut de 75 à 125 mg/m²/jour.

6. Emploi conforme à la revendication 1, dans lequel la période de traitement dure une, deux ou trois semaines.

7. Emploi conforme à la revendication 6, dans lequel la période de repos dure une semaine.

8. Emploi conforme à la revendication 7, dans lequel la période de traitement dure une semaine.

9. Emploi conforme à la revendication 8, dans lequel la dose quotidienne vaut de 75 à 125 mg/m²/jour.

10. Emploi conforme à la revendication 7, dans lequel la période de traitement dure trois semaines.

11. Emploi conforme à la revendication 10, dans lequel la dose quotidienne vaut dé 75 à 125 mg/m²/jour.
